# EUROPÄISCHE PATENTSCHRIFT

(11) **EP 1 931 405 B1**
(45) Veröffentlichungstag und Bekanntmachung des Hinweises auf die Patenterteilung: **14.11.2018**
(21) Anmeldenummer: 06791326.9
(22) Anmeldetag: 31.08.2006
(51) Int. Cl.: A61M 5/20, A61M 5/32

(54) **AUTOMATISCHE INJEKTIONSVORRICHTUNG FÜR ZWEI-KAMMER-AMPULLEN**
AUTOMATIC INJECTION DEVICE FOR TWO-CHAMBER AMPULLAS
DISPOSITIF A INJECTION AUTOMATIQUE POUR DES AMPOULES A DEUX CHAMBRES

(30) Priorität: 22.09.2005 DE 202005014958 U
(43) Veröffentlichungstag der Anmeldung: 18.06.2008
(73) Patentinhaber: Bayer Intellectual Property GmbH, 40789 Monheim (DE)
(72) Erfinder: WEBER, Wilfried, 72296 Schopfloch (DE)
(74) Vertreter: BIP Patents
(86) Internationale Anmeldenummer: PCT/DE2006/001512
(87) Internationale Veröffentlichungsnummer: WO 2007/033638

(56) Entgegenhaltungen:
- EP-A1- 1 518 575
- WO-A-2005/000384
- WO-A2-03/047663
- WO-A2-2005/011780
- FR-A- 2 616 221
- FR-E- 82 914
- US-B1- 6 387 078

## Beschreibung

Zur Behandlung einer Vielzahl von Krankheiten, die inzwischen eine große Verbreitung haben, wie z.B. der Zuckerkrankheit, muss der Patient selbständig sich die benötigte Menge eines Wirkstoffes/Arzneimittels mittels einer Spritze oder einer Karpule injizieren. Um dies sicherer und einfacher zu gestalten, sind eine Vielzahl von Injektionsvorrichtungen bekannt, die einen weitgehend automatischen Ablauf vom Einstechen der Nadel, Injizieren des Wirkstoffes und Zurückholen der Nadel beinhalten.

### Stand der Technik

Zur Verwendung von Einwegspritzen sind mehrere Vorrichtungen zur automatischen Injektion des in der Spritze enthaltenen Wirkstoffs bekannt; so zeigen z.B. die WO 2005/011780 oder die WO 99/56805 Injektionsvorrichtungen, die bei einfacher Bedienung einen vollautomatischen Ablauf der oben beschriebenen Vorgänge ermöglichen.

In jüngerer Zeit hat sich gezeigt, dass eine Verbesserung des Behandlungsergebnisses oder auch überhaupt eine Sicherung eines medizinischen Erfolges die Verwendung von Wirkstoffen erfordert, die unmittelbar vor der Injektion mit einem anderen Wirkstoff gemischt werden müssen, ein Beispiel hierfür ist Betaferon zur Mischung mit einer NaCI-Lösung. Zur Erreichung dieses Ziels sind die beiden Wirkstoffe in der Regel in einem Spritzenkörper mit zwei Kammern untergebracht, die voneinander getrennt sind und zwischen denen erst kurz vor der Injektion eine Verbindung hergestellt wird, über die dann die beiden Wirkstoffe (wenn möglich, für den Patienten sichtbar verfolgbar) gemischt werden.

Für diese speziellen Spritzenkörper, die im folgenden als Zwei-Kammer-Ampullen bezeichnet werden, sind Injektionsvorrichtungen bekannt, mit deren Hilfe eine Abfolge von Mischen, Einstechen und Injizieren ermöglicht wird (DE 600 11 853 T2). Die Steuerung dieser Abläufe erfolgt jedoch manuell und erfordert somit zur Handhabung große Aufmerksamkeit seitens des Patienten, zumal auch kein Zurückholen der Nadel nach abgeschossener Injektion vorgesehen ist.

Eine Verabreichungseinrichtung für eine Mehrkammerampulle zeigt die DE 103 40 585 A1. Hier ist der manuelle Aufwand zur Injektion ähnlich, zwei zylindrische, koaxial einander zugeordnete Gehäusehälften, in denen die Ampulle eingelegt ist, werden zum Mischen manuell zusammengedrückt bzw. geschraubt, bis eine Endposition erreicht ist; danach soll die Injektion die Injektion durch einen 'Verabreichungsmechanismus' durchgeführt werden, der nicht näher dargestellt ist. Ein automatisiertes Zurückholen der Nadel ist auch hier nicht vorgesehen.

WO 03/047663 A2 offenbart einen semi-automatischen Injektor für eine Zwei-Kammer-Spritze, bei dem die Vermischung des Inhalts der beiden Kammern durch manuelles Drehen Eindrehen eines Stössels erfolgt. Einstechen, Injektion und Rückholen der Spritze erfolgt dann nach Aktivierung automatisch.

EP 1 518 517 A1 offenbart einen automatischen Injektor für eine Zwei-Kammer-Spritze, wobei die Kraft für den Einstechhub und den Injektionshub jeweils durch einen Motor erzeugt werden.

US 6,387,078 B1 offenbart einen automatischen Injektor für eine Zwei-Kammer-Spritze, wobei die Zwei-Kammer-Spritze keinen Überströmkanal aufweist, sondern eine Versiegelung innerhalb des Spritzenkörper geöffnet wird damit Wirkstoff von der ersten zur zweiten Kammer gelangen kann. WO 2005/011780 A2 offenbart einen automatischen Injektor für eine Ein-Kammer-Spritze.

### Darstellung der Erfindung

Es ist Aufgabe der Erfindung, eine Injektionsvorrichtung für eine Zwei-Kammer-Ampulle durch Automatisierung der Abläufe derart weiterzubilden, dass bei einfacher mechanischer Gestaltung der Handhabungskomfort und die Sicherheit für den Patienten erhöht wird.

Eine weitere Aufgabe besteht darin, die bei Zwei-Komponenten-Wirkstoffen relativ kritischen Dosierungen für den einzelnen Patienten und auch die damit verbundenen Einstechtiefen an der Einstechstelle individuell zu regeln, so dass eine Optimierung für den jeweiligen Patienten sowohl hinsichtlich des injizierten Wirkstoffvolumens, als auch hinsichtlich der Einstichtiefe der Nadel erzielt werden kann.

Die erfindungsgemäße Injektionsvorrichtung löst diese Aufgabe mit den Merkmalen des Patentanspruchs 1.

Der grundlegende Erfindungsgedanke ist darin zu sehen, die Aufeinanderfolge der Hube ausschließlich durch einen einfachen Antriebsmechanismus, einen Seilzug, zu bewirken. Hierbei wird der vom Stössel durch Beaufschlagung der Zwei-Kammer-Ampulle durchgeführte Hub in einen vorausgehenden Mischhub und einen Injektionshub unterteilt, die durch den Einstechhub unterbrochen werden. Damit ist gewährleistet, dass das Einstechen der Nadel in die Einstichstelle erst erfolgt, wenn die Mischung der beiden Wirkstoffe erfolgt ist und vom Patienten gegebenenfalls durch ein Fenster als korrekt beurteilt worden ist.

Bevorzugte Ausgestaltung betreffen die Auslegung der Einstellelemente zur Ablaufsteuerung, die auch zur voneinander unabhängigen Einstellung der Einstechtiefe und der Einstellung des Injektionsvolumens des gemischten Wirkstoffes ausgebildet sind.

Weitere konstruktive Ausgestaltungen sind weiteren Unteransprüchen zu entnehmen.

### Kurze Beschreibung der Zeichnungen

Ein bevorzugtes Ausführungsbeispiel der Injektionsvorrichtung wird nun anhand von Zeichnungen näher erläutert. Es zeigen:
- Figur 1A:: Eine Seitenansicht der Injektionsvorrichtung in Ausgangsstellung,
- Figur 1B:: einen Schnitt durch die injektionsvorrichtung der Figur 1 in ihrer Grundstellung in ihrer Mittelebene,
- Figur 1C:: einen Schnitt durch die Injektionsvorrichtung in der Ebene S-S der Figur 1B,
- Figur 2A:: eine Seitenansicht der Injektionsvorrichtung bei der Durchführung des Mischhubes,
- Figur 2B:: eine Schnittdarstellung entsprechend Figur 2A,
- Figur 3A:: eine Seitenansicht der Injektionsvorrichtung beim Einstechhub,
- Figur 3B:: eine Schnittdarstellung gemäß Figur 3A,
- Figur 4A:: eine Seitendarstellung der Injektionsvorrichtung bei Injektionshub,
- Figur 4B:: eine Schnittdarstellung gemäß Figur 4A,
- Figur 5A:: eine Seitendarstellung der Injektionsvorrichtung zu Beginn des Nadelrückzugs,
- Figur 5B:: eine Schnittdarstellung gemäß Figur 5A,
- Figur 6A:: eine Seitenansicht der Injektionsvorrichtung nach Beendigung des Nadelrückzugs, und
- Figur 6B:: eine Schnittdarstellung gemäß Figur 6A,

### Beschreibung des Ausführungsbeispiels

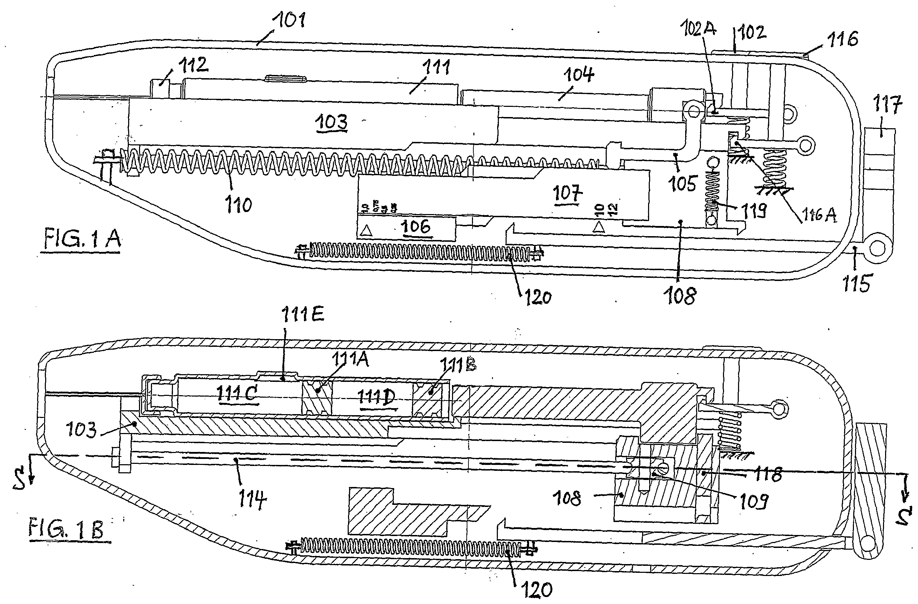

Zur Injektion des Wirkstoffes wird eine Zweikammer-Ampulle 111 verwendet. Eine solche Ampulle (Figur 1B) hat zwei Kolben 111A, 111B, dadurch ergeben sich zwei zunächst voneinander unabhängige Kammern 111C, 111D. In die erste, innere Kammer 111C, die der Kanüle 112 zugewandt ist, wird zum Beispiel Betaferon in Pulverform, in die zweite, äussere Kammer 111D eine NaCl - Lösung eingefüllt.

Wird nun ein Stössel 104 gegen den äußeren Kolben 111B gedrückt, verschiebt sich zunächst auch der innere Kolben 111A, da die NaCl - Lösung die Kraft des Stössels hydraulisch auf den inneren Kolben 111A überträgt. Sobald der innere Kolben 111A einen Überströmkanal 111E in Form einer nutförmigen Ausbuchtung im Mantel der Ampulle 111 überlaufen hat, bleibt dieser stehen und die NaCl- Lösung strömt über diesen Überströmkanal 111E in die innere Kammer 111C und mischt sich mit dem Betaferon. Nach dem Mischen erfolgt dann (nach dem Einstechhub) die Injektion durch das Weiterbewegen des Stössels 104.

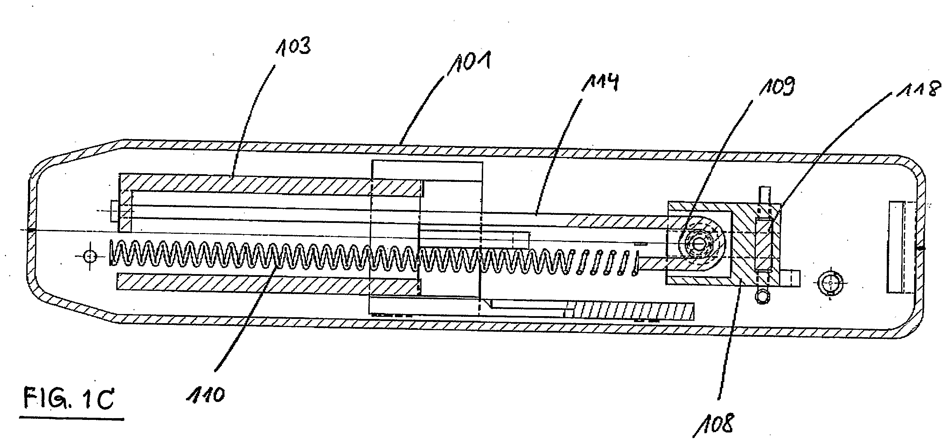

Figur 1A zeigt eine Aufsicht, Figur 1B einen Schnitt in der Ausgangsstellung der Injektionsvorrichtung, Figur 1C einen weiteren Schnitt in der Ebene S-S.

Sämtliche Bauteile befinden sich in einem Gehäuse 101, das aus zwei wannenförmigen Halbschalen besteht. Die Bewegungsbauteile sind hierbei in der Injektionsvorrichtung parallel zur Längsachse der Nadel verschiebbar gehalten. Die Bauteile sind wie folgt einander zugeordnet:
Eine Zweikammer-Ampulle 111 ist in einer Aufnahme 103 gehalten. Ein Stössel 104, an dessen hinterem Ende ein Steuerhebel 105 angelenkt ist, ist an einem Rasthaken 102A einer federbeaufschlagten ersten Taste 102 gehalten. Die Aufnahme 103 ist an einem Rasthaken 116A einer federbeaufschlagten zweiten Taste 116 gehalten.

Auf die Aufnahme 103 wirkt das Ende eines Zugseils 114, welches über eine in einem Schlitten 108 gelagerte Rolle 109 umgelenkt wird, und mit einer Zugfeder 110 verbunden ist, die am Gehäuse 101 befestigt ist. Die Zugfeder 110 übt somit einen Zug auf die Aufnahme 103 in entgegengesetzter Richtung zur Einstichstelle aus. Die Aufnahme 103 kann sich jedoch nicht axial verschieben, da sie vom Rasthaken 116A an der zweiten Taste 116 gehalten wird.

Durch die Umlenkung des Zugseiles 114 über die Rolle 109 entsteht eine Kraft auf den Schlitten 108 in Richtung der Einstichstelle. Der Schlitten 108 bleibt jedoch in seiner Position, da er über einen im Schlitten 108 senkrecht zur Injektionsrichtung verschiebbar gelagerten von einer Mitnehmerfeder 119 beaufschlagten Mitnehmer 118 am Stössel 104 anliegt und der Stössel 104 vom Rasthaken 102A an der ersten Taste 102 gehalten wird.

Dem Steuerhebel 105 ist ein erster Einstellschieber 107 zugeordnet, in dem ein zweiter Einstellschieber 106 verschiebbar gelagert ist. Der Einstellschieber 106 dient zur Entkoppelung des Schlittens 108 vom Stössel 104. Die Einstellschieber 106,107 sind als verschiebbar gelagerte Anschlagelemente zur Einstellung von Einstechtiefe und Injektionsvolumen ausgebildet, wie dies weiter unten noch erläutert wird.

Ein Rückzuggriff 117, der mit einer Zugstange 115 verbunden ist, dient zur Herstellung dieser Ausgangslage. Die Zugstange 115 wird durch eine Rückzugfeder 120 beaufschlagt.

### Beschreibung des Ablaufes

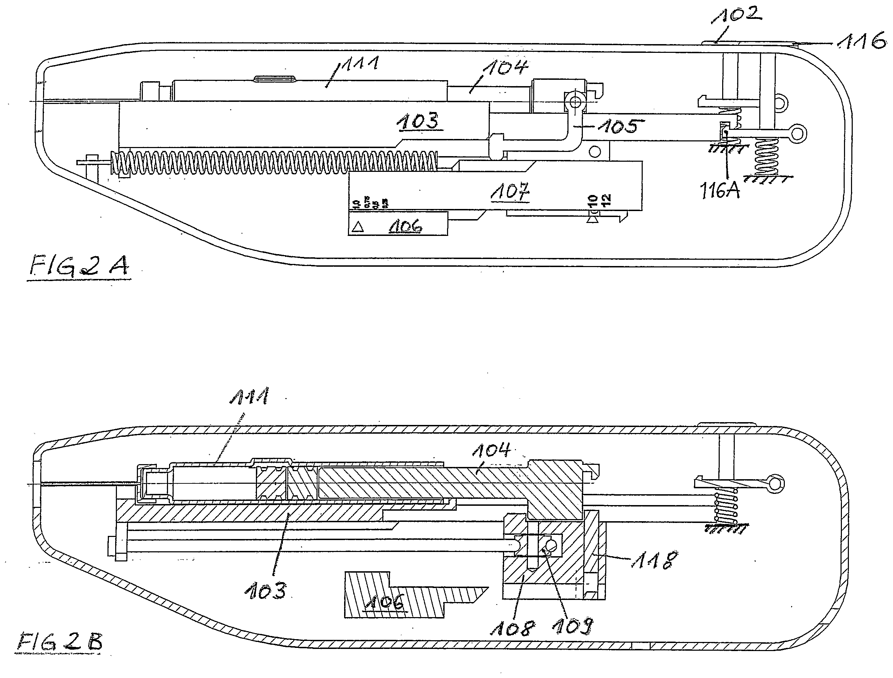

Wird die erste Taste 102 betätigt, kommt der Rasthaken 102A außer Eingriff, der Stössel 104 wird freigegeben und bewegt sich in Richtung Einstichstelle, bis die Vorderkante des Steuerhebels 105 an der Aufnahme 103 anliegt. Auf diese Weise wird der äußere Kolben 111B der Ampulle 111 beaufschlagt, bewegt sich nach vorne und führt einen Mischhub H0 durch. Dieser Mischhub dient dem Mischen der NaCI-Lösung mit dem Betaferon wie oben beschrieben (Figur 2A, Figur 2B). Durch ein Sichtfenster im Gehäuse 101 kann die Vermischung des Betaferons mit der NaCI-Lösung kontrolliert werden.

Da das freie Ende des Steuerhebels 105 andererseits auf dem zweiten Einstellschieber 107 gleitet und dort aufliegt, kann er an dieser Stelle nicht durch Verschwenken nach unten ausweichen; somit wird die Zugkraft der Zugfeder 110 in Richtung Einstichstelle vom Schlitten 108 über den Stössel 104 auf die Aufnahme 103 übertragen. Die Aufnahme 103 bleibt jedoch in ihrer Lage, da sie vom Rasthaken 116A der Taste 116 arretiert ist.

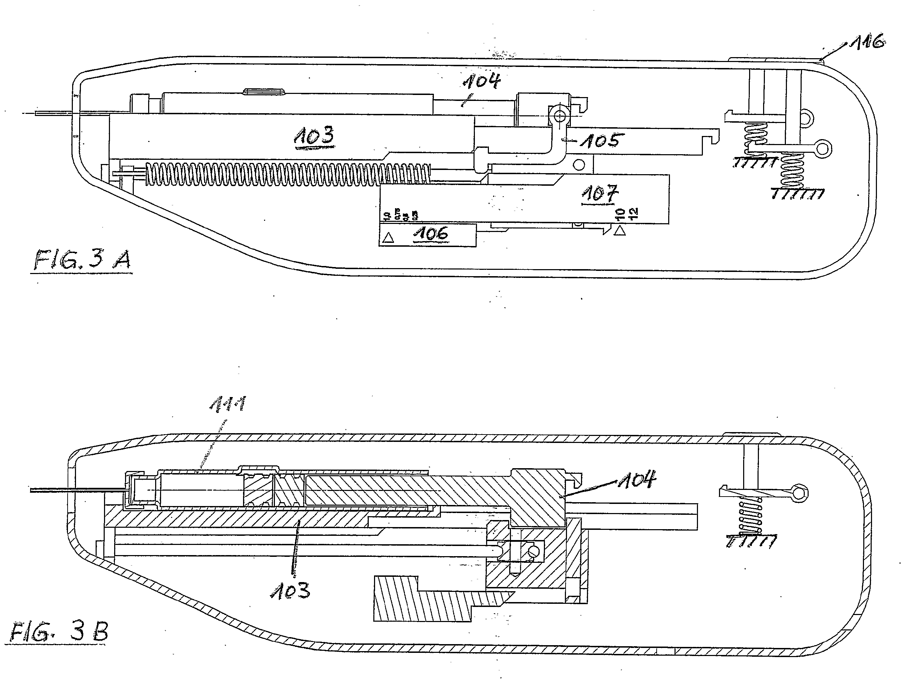

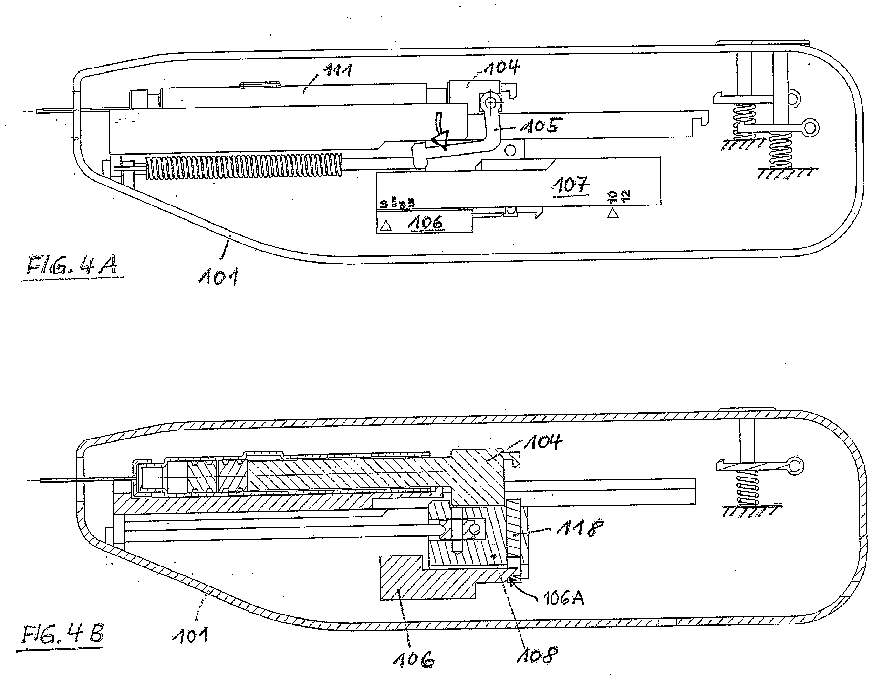

Wird nun die zweite Taste 116 betätigt, kommt der Rasthaken 116A außer Eingriff und die Aufnahme 103 wird freigegeben; dadurch bewegen sich der Stössel 104 und die Aufnahme 103 unter der Wirkung der Zugfeder 110 gemeinsam in Richtung Einstichstelle. Die Nadel wird eingestochen (Figur 3A,3B), der Einstechhub H1 wird durchgeführt (Figur 4A, 4B)

Ist die gewünschte Einstechtiefe erreicht, kann der Steuerhebel 105 nach unten schwenken (Pfeil in Figur 4A), da er nicht mehr durch den ersten Einstellschieber 107 wegen dessen zurückspringender Oberfläche daran gehindert wird. Es erfolgt somit keine Kraftübertragung mehr vom Stössel 104 auf die Aufnahme 103, die Aufnahme 103 verbleibt in ihrer Position, nur der Stössel 104 bewegt sich weiter zur Einstichstelle hin, d. h. die Injektion des Arzneimittels erfolgt, der Injektionshub H2 wird durchgeführt.

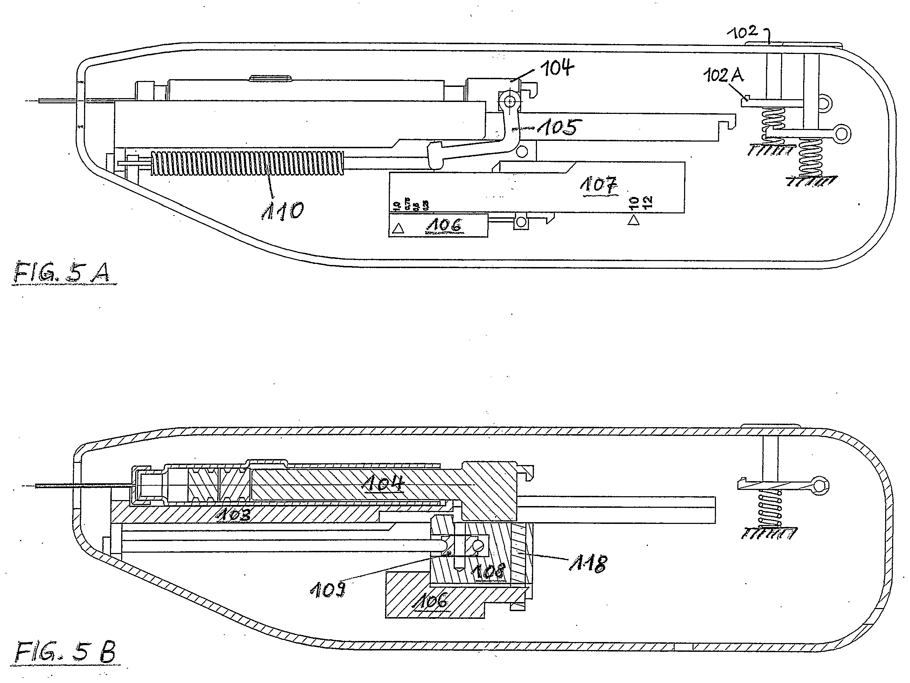

Erreicht der im Schlitten 108 verschiebbar gelagerte Mitnehmer 118 die Rampe 106A des zweiten Einstellschiebers 106 (Figur 4B), wird der Mitnehmer 118 nach unten gezogen und somit der Schlitten 108 vom Stössel 104 entkoppelt, d. h. zu diesem Zeitpunkt wird die Injektion beendet (Figur 5B).

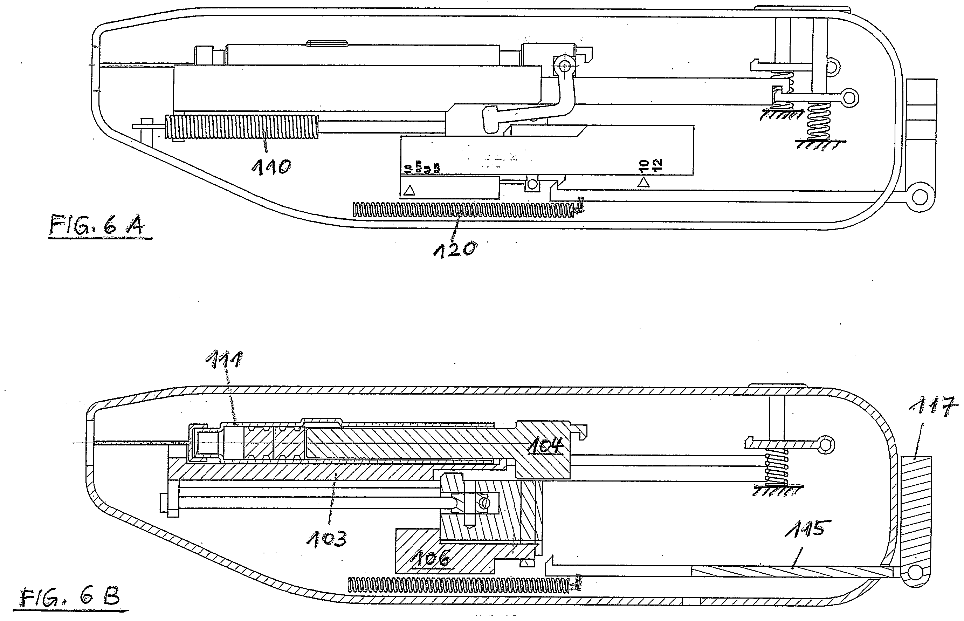

Der Schlitten 108 steht nun am Einstellschieber 106 an. Da der Einstellschieber 106 über den ersten Einstellschieber 107 formschlüssig am Gehäuse 101 gehalten wird, wirkt nun die Zugkraft der Zugfeder 110 (die am Gehäuse 101 befestigt ist) über die Rolle 109 auf die Aufnahme 103, die dadurch zurückgezogen wird und damit die Nadel aus der Einstichstelle zieht (Figur 6A, 6B), der Rückholhub H3 wird durchgeführt.

Durch Herunterklappen des Rückzuggriffes 117, der mit der Zugstange 115 verbunden ist, und Herausziehen der Zugstange 115 werden der Schlitten 108 und alle anderen Elemente wieder in ihre Ausgangslage (Figur 1A, 1B) zurückgezogen. Die Zugstange 115 wird durch die Rückzugfeder 120 wieder eingezogen.

Die Ampulle kann nun entnommen werden.

Das Injektionsvolumen und die Einstechtiefe lassen sich wie folgt einstellen:
Der erste Einstellschieber 107 ist im Gehäuse 101 axial verschiebbar gelagert, er hat in diesem Beispiel 2 Rastpositionen (10 und 12 mm, im Beispiel auf 10 mm eingestellt). Diese Rastpositionen sind dem Einstechhub H1 zugeordnet, da die axiale Lage des Einstellschiebers 107 den Weg bestimmt, bis der Steuerhebel 105 den Stössel 104 von der Aufnahme 103 entkoppelt (Figur 2A).

Im ersten Einstellschieber 107 ist der zweite Einstellschieber 106 ebenfalls axial verschiebbar mit im Beispiel 4 Rastpositionen gelagert (1.0; 0,75; 0,5; 0,25, im Beispiel auf 1,0 eingestellt). Diese Rastpositionen sind dem Injektionshub H2 zugeordnet, da die axiale Lage des Einstellschiebers 106 den Weg bestimmt, bis der Stössel 104 vom Schlitten 108 entkoppelt wird (Figur 5A,5B) und der Nadelrückzug erfolgt.

Soll nun z. B. eine Einstechtiefe von 12 mm eingestellt werden, muss gegenüber dem dargestellten Zustand der erste Einstellschieber 107 um 2 mm in Richtung der Einstechstelle in die neue Rastposition am Gehäuse 101 verschoben werden. Da der zweite Einstellschieber 106 mit dem ersten Einstellschieber 107 in der Position 1,0 verrastet ist, verschiebt sich auch dieser um 2 mm zur Einstechstelle hin, d. h., die Einstellung einer anderen Einstechtiefe wirkt sich nicht auf die Einstellung des Injektionsvolumens aus. Ebenso wirkt sich die Einstellung des Injektionsvolumens nicht auf Einstechtiefe aus; die Einstellungen von Einstechhub H1 und Injektionshub H2 sind unabhängig voneinander.

### Bezugszeichen

- Gehäuse: 101
- erste Taste: 102
- Rasthaken: 102A
- Aufnahme: 103
- Stössel: 104
- Steuerhebel: 105
- erster Einstellschieber: 107
- zweiter Einstellschieber: 106
- Rampe: 106A
- Schlitten: 108
- Rolle: 109
- Feder: 110
- Ampulle: 111
- Kanüle: 112
- Zugseil: 114
- Zugstange: 115
- zweite Taste: 116
- Rasthaken: 116A
- Rückzuggriff: 117
- Mitnehmer: 118
- Mitnehmerfeder: 119
- Rückzugfeder: 120

## Patentansprüche

1. Injektionsvorrichtung zur Aufnahme und Betätigung einer Zwei-Kammer- Ampulle (111), die einen Ampullenkörper mit einem inneren Kolben (111A), einem äußeren Kolben (111B) einer Injektionsnadel an einem Ende des Ampullenkörpers sowie einer inneren Kammer (111C) zwischen dem inneren Kolben (111A) und der Injektionsnadel und einer äußeren Kammer (111D) zwischen dem inneren Kolben (111A) und dem äußeren Kolben (111B) und einen Überströmkanal im Bereich der inneren Kammer (111C) aufweist,
mit Bauteilen, durch deren Relativbewegung die Kolben (111A, 111B) der Zwei-Kammer-Ampulle (111) zur Mischung von in den beiden Kammern (111C, 111D) befindlichen Wirkstoffen verschoben werden, bis der innere Kolben (111A) den Überströmkanal (111E) der Zwei-Kammer-Ampulle erreicht und stehen bleibt, worauf der äußere Kolben (111B) den in der äußeren Kammer (111D) befindlichen ersten Wirkstoff in die die innere Kammer (111C) mit dem zweiten Wirkstoff fördert,
sowie mit Vorrichtungen zur Injektion des derart gemischten Produkts,
**dadurch gekennzeichnet, dass**
in einem Gehäuse (101) eine Aufnahme (103) gehalten ist, in die die Zwei-Kammer-Ampulle (111) einlegbar und fixierbar ist;
die Aufnahme (103) mittels eines Schlittens (108) und eines mit dem Schlitten (108) gekoppelten Stössels (104) verschiebbar ist;
in der Aufnahme (103) der Stössel (104) verschiebbar gehalten ist, der die Kolben (111A, 111B) beaufschlagt;
ein Zugseil (114) vorgesehen ist, das über eine am Schlitten (108) gelagerte Rolle (109) umgelenkt wird und dessen eines Ende mit der Aufnahme (103) und dessen anderes Ende mit einer Zugfeder (110) verbunden ist, die am Gehäuse (101) gehalten ist, und das Zugseil eine Kraft auf den Schlitten (108) in Richtung einer Einstichstelle für die Injektionsnadel ausübt;
erste automatisch und/oder manuell betätigbare Einrichtungen zur lösbaren Arretierung des Stössels (104) zur Einleitung eines Mischhubs vorgesehen sind; und
zweite automatisch und/oder manuell betätigbare Einrichtungen zur lösbaren Arretierung der Aufnahme (103) zur Einleitung eines Einstechhubs nach dem Mischhub vorgesehen sind, wenn mittels des Schlittens (108) über den Stössel (104) Kraft in Richtung Einstichstelle auf die Aufnahme (103) übertragen wird.

2. Injektionsvorrichtung nach Anspruch 1, **dadurch gekennzeichnet, dass** die ersten Einrichtungen aus einem Rasthaken (102A) einer federbeaufschlagten, ersten Taste (102) am Gehäuse (101) bestehen, der am Stössel (104) angreift.

3. Injektionsvorrichtung nach Anspruch 1, **dadurch gekennzeichnet, dass** die zweiten Einrichtungen aus einem Rasthaken (116A) an mindestens einer federbeaufschlagten Taste (116) am Gehäuse (101) bestehen, der an der Aufnahme (103) angreift.

4. Injektionsvorrichtung nach Anspruch 1, **dadurch gekennzeichnet, dass** das Zugseil (114) über den Schlitten (108) und die Rolle (109) bei einem auf den Injektionshub folgenden Rückholhub die Aufnahme (103) mit der Ampulle und der Injektionsnadel aus der Einstichstelle zieht.

5. Injektionsvorrichtung nach Anspruch 1, **dadurch gekennzeichnet, dass** dritte Einrichtungen zur Koppelung des Stössels (104) mit der Aufnahme (103) vorgesehen sind, die den Stössel (104) zur Durchführung des Einstechhubs mit der Aufnahme (103) koppeln und zur Durchführung eines Injektionshubs entkoppeln.

6. Injektionsvorrichtung nach Anspruch 5, **dadurch gekennzeichnet, dass** die dritten Einrichtungen aus einem Steuerhebel (105) bestehen, dessen eines Ende am Stössel (104) angelenkt ist und dessen anderes Ende die Aufnahme (103) beaufschlagt.

7. Injektionsvorrichtung nach Anspruch 1, **dadurch gekennzeichnet, dass** die Kopplung des Schlittens (108) mit dem Stössel (104) mittels eines federbeaufschlagten Mitnehmers (118) erfolgt, der im Schlitten (108) gelagert ist und in seiner Koppelposition am Stössel (104) angreift.

8. Injektionsvorrichtung nach Anspruch 1, **dadurch gekennzeichnet, dass** sie einen ersten Einstellschieber (107) enthält, der eine Einstellung des Wegs des Einstechhubs und damit der Einstechtiefe bewirkt.

9. Injektionsvorrichtung nach Anspruch 6 und 8, **dadurch gekennzeichnet, dass** der erste Einstellschieber (107) als zwischen zwei Endpositionen beliebig verschiebbar gelagertes Anschlagelement ausgebildet ist, dessen Position über den Steuerhebel (105) den Weg der Aufnahme (103) und damit die Länge des Einstechhubs bestimmt.

10. Injektionsvorrichtung nach Anspruch 1, **dadurch gekennzeichnet, dass** sie einen zweiten Einstellschieber (106) enthält, der durch den Weg des Stössels (104) eine Einstellung der Länge des Injektionshubs und damit der Injektionsmenge des gemischten Wirkstoffes bewirkt.

11. Injektionsvorrichtung nach Anspruch 10, **dadurch gekennzeichnet, dass** der
zweite Einstellschieber (106) als ein zwischen mindestens zwei Positionen relativ zum ersten Einstellschieber (107) verschiebbar gelagertes Anschlagelement für die Aufnahme (103) ausgebildet ist.

12. Injektionsvorrichtung nach den Ansprüchen 8 bis 11, **dadurch gekennzeichnet, dass** einer der Einstellschieber verschiebbar oder drehbar, mittelbar oder unmittelbar, im jeweils anderen Einstellschieber gelagert ist, so dass Einstechtiefe und Injektionsvolumen unabhängig voneinander einstellbar sind.

13. Injektionsvorrichtung nach Anspruch 1, **dadurch gekennzeichnet, dass** das Gehäuse (101) aus zwei Halbschalen besteht.

14. Injektionsvorrichtung nach Anspruch 13, **dadurch gekennzeichnet, dass** zumindest eine der Halbschalen ein Sichtfenster zur Kontrolle der Mischung der beiden Substanzen in der vorderen Kammer beim Mischhub aufweist.

## Claims

1. Injection device for receiving and actuating a two-chamber ampoule (111) which has an ampoule body with an inner piston (111A), an outer piston (111B) of an injection needle at one end of the ampoule body and also an inner chamber (111C) between the inner piston (111A) and the injection needle and an outer chamber (111D) between the inner piston (111A) and the outer piston (111B) and a transfer channel in the region of the inner chamber (111C),
with components whose relative movement causes the pistons (111A, 111B) of the two-chamber ampoule (111) to be displaced, for the purpose of mixing active substances located in the two chambers (111C, 111D), until the inner piston (111A) reaches the transfer channel (111E) of the two-chamber ampoule and comes to a halt, whereupon the outer piston (111B) conveys the first active substance, located in the outer chamber (111D), into the inner chamber (111C) containing the second active substance,
and with devices for injecting the product that is thus mixed,
**characterized in that**
a receptacle (103), into which the two-chamber ampoule (111) can be placed and fixed, is held in a housing (101);
the receptacle (103) is displaceable by means of a carriage (108) and by means of a tappet (104) coupled to the carriage (108);
the tappet (104), which acts on the pistons (111A, 111B), is held displaceably in the receptacle (103) ;
a traction cable (114) is provided, which is deflected via a roller (109) mounted on the carriage (108), and one end of which is connected to the receptacle (103) and the other end of which is connected to a tension spring (110), which is held on the housing (101), and the traction cable exerts a force on the carriage (108) in the direction of an insertion site for the injection needle;
first automatically and/or manually actuatable devices are provided for releasably locking the tappet (104) in order to initiate a mixing stroke; and
second automatically and/or manually actuatable devices are provided for releasably locking the receptacle (103) in order to initiate an insertion stroke after the mixing stroke when, by means of the carriage (108), force is transmitted via the tappet (104) to the receptacle (103) in the direction of the insertion site.

2. Injection device according to Claim 1, **characterized in that** the first devices comprise a locking hook (102A) of a spring-loaded, first push-button (102) on the housing (101), which locking hook (102A) acts on the tappet (104).

3. Injection device according to Claim 1, **characterized in that** the second devices comprise a locking hook (116A) on at least one spring-loaded push-button (116) on the housing (101), which locking hook (116A) acts on the receptacle (103) .

4. Injection device according to Claim 1, **characterized in that** the traction cable (114) pulls the receptacle (103) with the ampoule and the injection needle out from the insertion site, by means of the carriage (108) and the roller (109), during a return stroke following the injection stroke.

5. Injection device according to Claim 1, **characterized in that** third devices are provided for coupling the tappet (104) to the receptacle (103), said third devices coupling the tappet (104) to the receptacle (103) in order to carry out the insertion stroke and uncoupling them in order to carry out an injection stroke.

6. Injection device according to Claim 5, **characterized in that** the third devices comprise a control lever (105), one end of which is hinged on the tappet (104), and the other end of which acts on the receptacle (103).

7. Injection device according to Claim 1, **characterized in that** the coupling of the carriage (108) to the tappet (104) is effected by means of a spring-loaded driver (118) which is mounted in the carriage (108) and, in its coupling position, acts on the tappet (104).

8. Injection device according to Claim 1, **characterized in that** it incorporates a first adjusting slide (107), which effects an adjustment of the path of the insertion stroke and, therefore, of the insertion depth.

9. Injection device according to Claims 6 and 8, **characterized in that** the first adjusting slide (107) is designed as a stop element which is mounted so as to be displaceable between two end positions as desired, and whose position determines, via the control lever (105), the path of the receptacle (103) and thus the length of the insertion stroke.

10. Injection device according to Claim 1, **characterized in that** it incorporates a second adjusting slide (106) which, via the path of the tappet (104), effects an adjustment of the length of the injection stroke and thus of the injection quantity of the mixed active substance.

11. Injection device according to Claim 10, **characterized in that** the second adjusting slide (106) is designed as a stop element for the receptacle (103), said stop element being mounted so as to be displaceable relative to the first adjusting slide (107) between at least two positions.

12. Injection device according to Claims 8 to 11, **characterized in that** one of the adjusting slides is mounted displaceably or rotatably, indirectly or directly, in the respective other adjusting slide, such that insertion depth and injection volume are adjustable independently of each other.

13. Injection device according to Claim 1, **characterized in that** the housing (101) is composed of two half shells.

14. Injection device according to Claim 13, **characterized in that** at least one of the half shells has a viewing window for monitoring the mixing of the two substances in the front chamber during the mixing stroke.

## Revendications

1. Dispositif d'injection pour recevoir et actionner une ampoule à deux chambres (111), qui présente un corps d'ampoule avec un piston intérieur (111A), un piston extérieur (111B), une aiguille d'injection à une extrémité du corps d'ampoule ainsi qu'une chambre intérieure (111C) entre le piston intérieur (111A) et l'aiguille d'injection, et une chambre extérieure (111D) entre le piston intérieur (111A) et le piston extérieur (111B), et qui présente un canal de trop-plein dans la région de la chambre intérieure (111C),
avec des composants dont le mouvement relatif permet de déplacer les pistons (111A, 111B) de l'ampoule à deux chambres (111) pour mélanger les substances actives se trouvant dans les deux chambres (111C, 111D), jusqu'à ce que le piston intérieur (111A) atteigne le canal de trop-plein (111E) de l'ampoule à deux chambres et reste en position, sur quoi le piston extérieur (111B) refoule la première substance active se trouvant dans la chambre extérieure (111D) dans la chambre intérieure (111C) comprenant la deuxième substance active,
et comprenant des dispositifs pour l'injection du produit ainsi mélangé,
**caractérisé en ce que**
un logement (103) est prévu dans un boîtier (101), dans lequel peut être introduite et fixée l'ampoule à deux chambres (111) ;
le logement (103) peut être déplacé au moyen d'un chariot (108) et d'un poussoir (104) accouplé au chariot (108) ;
le poussoir (104) est maintenu de manière déplaçable dans le logement (103) et sollicite le piston (111A, 111B) ;
un câble de traction (114) est prévu, lequel est dévié par le biais d'une poulie (109) supportée sur le chariot (108), l'une de ses extrémités étant connectée au logement (103) et son autre extrémité étant connectée à un ressort de traction (110) qui est retenu sur le boîtier (101) et le câble de traction exerce une force sur le chariot (108) dans la direction d'une zone de piqûre pour l'aiguille d'injection ;
des premiers dispositifs pouvant être actionnés automatiquement et/ou manuellement pour le blocage libérable du poussoir (104) sont prévus pour amorcer une course de mélange ; et
des deuxièmes dispositifs pouvant être actionnés automatiquement et/ou manuellement pour le blocage libérable du logement (103) sont prévus pour amorcer une course de piqûre après la course de mélange lorsqu'une force est transmise au logement (103) au moyen du chariot (108) par le biais du poussoir (104) dans la direction de la zone de piqûre.

2. Dispositif d'injection selon la revendication 1, **caractérisé en ce que** les premiers dispositifs se composent d'un crochet d'encliquetage (102A) d'une première touche sollicitée par ressort (102) sur le boîtier (101), qui s'engage avec le poussoir (104) .

3. Dispositif d'injection selon la revendication 1, **caractérisé en ce que** les deuxièmes dispositifs se composent d'un crochet d'encliquetage (116A) au niveau d'au moins une touche sollicitée par ressort (116) sur le boîtier (101), qui s'engage avec le logement (103).

4. Dispositif d'injection selon la revendication 1, **caractérisé en ce que** le câble de traction (114), dans le cas d'une course de rappel suivant la course d'injection, tire, par le biais du chariot (108) et de la poulie (109), le logement (103) avec l'ampoule et l'aiguille d'injection hors de la zone de piqûre.

5. Dispositif d'injection selon la revendication 1, **caractérisé en ce que** des troisièmes dispositifs pour l'accouplement du poussoir (104) au logement (103) sont prévus, lesquels accouplent le poussoir (104) au logement (103) pour effectuer la course de piqûre, et les désaccouplent pour effectuer une course d'injection.

6. Dispositif d'injection selon la revendication 5, **caractérisé en ce que** les troisièmes dispositifs se composent d'un levier de commande (105) dont une extrémité est articulée au poussoir (104) et dont l'autre extrémité sollicite le logement (103) .

7. Dispositif d'injection selon la revendication 1, **caractérisé en ce que** l'accouplement du chariot (108) au poussoir (104) s'effectue au moyen d'un dispositif d'entraînement sollicité par ressort (118), qui est supporté dans le chariot (108) et qui s'engage dans sa position d'accouplement avec le poussoir (104).

8. Dispositif d'injection selon la revendication 1, **caractérisé en ce qu'**il contient un premier coulisseau d'ajustement (107) qui effectue un ajustement du parcours de la course de piqûre et donc de la profondeur de piqûre.

9. Dispositif d'injection selon les revendications 6 et 8, **caractérisé en ce que** le premier coulisseau d'ajustement (107) est réalisé sous la forme d'un élément de butée supporté de manière déplaçable de manière quelconque entre deux positions d'extrémité, dont la position définit, par le biais du levier de commande (105), le parcours du logement (103) et donc la longueur de la course de piqûre.

10. Dispositif d'injection selon la revendication 1, **caractérisé en ce qu'**il contient un deuxième coulisseau d'ajustement (106) qui provoque, par le parcours du poussoir (104), un ajustement de la longueur de la course d'injection et donc de la quantité d'injection de la substance active mélangée.

11. Dispositif d'injection selon la revendication 10, **caractérisé en ce que** le deuxième coulisseau d'ajustement (106) est réalisé sous la forme d'un élément de butée pour le logement (103), supporté de manière déplaçable entre au moins deux positions par rapport au premier coulisseau d'ajustement (107).

12. Dispositif d'injection selon les revendications 8 à 11, **caractérisé en ce que** l'un des coulisseaux d'ajustement est supporté de manière déplaçable ou rotative, directement ou indirectement, dans l'autre coulisseau d'ajustement respectif, de telle sorte que la profondeur de piqûre et le volume d'injection puissent être ajustés indépendamment l'un de l'autre.

13. Dispositif d'injection selon la revendication 1, **caractérisé en ce que** le boîtier (101) se compose de deux demi-coques.

14. Dispositif d'injection selon la revendication 13, **caractérisé en ce qu'**au moins l'une des demi-coques présente une fenêtre d'inspection pour contrôler le mélange des deux substances dans la chambre avant lors de la course de mélange.
